(19) Europäisches Patentamt
European Patent Office
Office européen des brevets

(11) EP 1 336 403 A1

(12) EUROPÄISCHE PATENTANMELDUNG

(43) Veröffentlichungstag:
**20.08.2003 Patentblatt 2003/34**

(51) Int Cl.[7]: **A61K 7/48**, A61K 7/02

(21) Anmeldenummer: **03002727.0**

(22) Anmeldetag: **06.02.2003**

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**AL LT LV MK RO**

(30) Priorität: **15.02.2002 DE 10206664**

(71) Anmelder: **Henkel Kommanditgesellschaft auf Aktien**
**40589 Düsseldorf-Holthausen (DE)**

(72) Erfinder:
- **Yücel, Sevda**
  **40591 Düsseldorf (DE)**
- **Waldmann-Laue, Marianne, Dr.**
  **40789 Monheim (DE)**
- **Wadle, Armin, Dr.**
  **40699 Erkath (DE)**

# (54) 6,7-disubstituierte 2,2-Dialkylchromane oder -chromene als Entzündungshemmer

(57) Die Erfindung betrifft die Verwendung von 6,7-disubstituierten 2,2-Dialkylchromanen oder -chromenen der allgemeinen Formeln (I) oder (II),

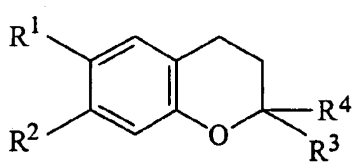

(I)

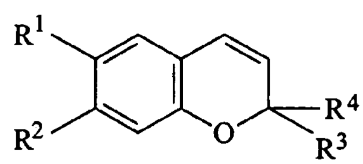

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, zur Herstellung topischer kosmetischer oder pharmazeutischer Zusammensetzungen mit entzündungshemmender Wirkung.

Die Emulgatoren sind in einer Menge von 0,2 - 10 Gew.-%, bevorzugt 0,5 - 8 Gew.-% und besonders bevorzugt 1 - 7 Gew.-%, jeweils bezogen auf die gesamte Zusammensetzung, enthalten.

EP 1 336 403 A1

Printed by Jouve, 75001 PARIS (FR)

## Beschreibung

Die Erfindung betrifft topische kosmetische und pharmazeutische Zusammensetzungen mit entzündungshemmender Wirkung, die als Wirkstoff 6,7-disubstituierte 2,2-Dialkylchromane oder -chromene enthalten.

Bekannte 2,2-Dialkylchroman-Derivate sind vor allem Tocopherole und Tocotrienole. Die Tocopherole sind in 2-Position mit einem Methylrest und mit einem gesättigten 4',8',12'-Trimethyltridecyl-Rest substituiert und weisen in 5-, 7- und 8-Position entweder ein Wasserstoffatom oder eine Methylgruppe, auf (α-, β-, γ-, δ-, $\zeta_2$- und η-Tocopherole). Die Tocotrienole, auch als ε-Tocopherole bezeichnet, unterscheiden sich von den vorgenannten Tocopherolen dadurch, dass in 2-Position an Stelle des gesättigten 4',8',12'-Trimethyltridecyl-Restes ein dreifach ungesättigter 4',8',12'-Trimethyltridecatrienyl-Rest aus drei Isoprenyl-Einheiten vorhanden ist. Neben ihrer Anwendung als Vitamin E wirken Tocopherole als Antioxidantien in Fetten und Ölen. Darüber hinaus besitzen Tocopherole eine Vielzahl von günstigen physiologischen Eigenschaften, z. B. Reduzierung von Muskelschäden, die auf oxidativen Streß während körperlicher Höchstleistung zurückzuführen sind, Verminderung des Risikos der Kataraktbildung, Verminderung des oxidativen Stresses bei Rauchern sowie anticarcinogene Effekte. Kosmetisch zeigen sie eine protektive Wirkung gegen Hautschäden und Hautalterung und schützen das Haar vor Witterungseinflüssen. Aus der PCT-Druckschrift WO 94/04128 A1 ist die Verwendung von Radikalfängern, darunter Tocopherole und ihre Derivate, zur Behandlung und Prophylaxe der durch UV-B-Strahlung induzierten Immunsuppression bekannt. Aus der Patentschrift US 5,593,682 sind Hautbehandlungsmittel mit 20 Gewichts-% Vitamin E bekannt, die Entzündungen, Schwellungen, Juckreiz und Narben reduzieren und die Wundheilung begünstigen. Die Patentschrift US 4,144,325 offenbart topische Zusammensetzungen zur Verhütung von durch UV-Strahlung verursachten Erythemen, die neben diversen UV-Filtersubstanzen höhere Mengen an Tocopherolen enthalten, um die Absorption dieser UV-Filter zu steigern. Die Patentschrift US 5,545,398 offenbart Tocotrienole als Wirkstoffe in topischen Zusammensetzungen zur Behandlung und Vorbeugung von Entzündungen, trockener Haut, Hautalterung, Psoriasis, Dermatitis und Verbrennungen und zum Schutz und zur Behandlung des Haares vor Sonnenlicht und chemischer Schädigung.

Neben den erwähnten Tocopherolen und Tocotrienolen sind weitere 2,2-Dialkylchroman-Derivate als kosmetische Wirkstoffe bekannt. Die Offenlegungsschrift EP 1 174 140 A1 offenbart Chromanolglycoside als Wirkstoff, der effektiv aktiven Sauerstoff und freie Radikale eliminiert, die die Ursache von durch UV-Licht hervorgerufenen lokalen Hautentzündungen darstellen. Explizit offenbart ist 2,5,7,8-Tetramethylchroman-6-ol, das in 2-Position mit einem Glucopyranosylmethyl-, Galactopyranosylmethyl-, Fructofuranosylmethyl- oder Mannopyranosylmethyl-Rest substituiert ist. Die Patentschrift US 5,811,083 offenbart das Amid aus 3,4-Dihydro-6-hydroxy-2,5,7,8-tetramethyl-1-benzopyran-2-carbonsäure (Trolox C) und Cysteamin als Antioxidans gegen Lipidperoxidation, wodurch es zur Behandlung von Hautatrophien, z.B. bei trockener Haut, Schuppen, Psoriasis, Altersflecken, entzündlichen Dermatosen und altersbedingten Hautveränderungen geeignet sein soll. Die Offenlegungsschrift EP 655 239 A1 offenbart die Verwendung von 2,2-Dimethylchromanen und -chromenen, die in 6-und 7-Position mit OH-, $CH_3O$- oder $CF_3CH_2O$-Gruppen substituiert sein können, als Antioxidantien in Doxorubicin-haltigen Liposomen. Daran anknüpfend, offenbart die Offenlegungsschrift EP 1 002 533 A1 die Verwendung der genannten 2,2-Dimethylchromane und -chromene als Antioxidantien in Zusammensetzungen zur topischen oder oralen Verabreichung. Die Antioxidantien erwiesen sich im Hauttest als nichtreizend, eine entzündungshemmende Wirkung ist allerdings nicht offenbart.

Überraschend und für den Fachmann nicht vorhersehbar wurde nun gefunden, dass topische Zusammensetzungen, die ausgewählte 6,7-disubstituierte 2,2-Dialkylchromane oder -chromene enthalten, eine entzündungshemmende Wirkung aufweisen. Insbesondere wurde gezeigt, dass topische Zusammensetzungen, die ausgewählte 6,7-disubstituierte 2,2-Dialkylchromane oder -chromene enthalten, in der Epidermis eine durch simuliertes Sonnenlicht ausgelöste Ausschüttung des Entzündungsmediators Interleukin-1α hemmen.

Gegenstand der vorliegenden Erfindung ist die Verwendung von 6,7-disubstituierten 2,2-Dialkylchromanen oder -chromenen der allgemeinen Formeln (I) oder (II),

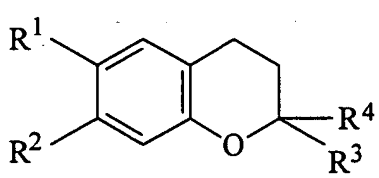

(I)

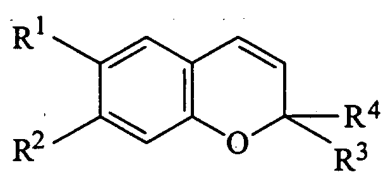

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, zur Herstellung topischer kosmetischer oder pharmazeutischer Zusammensetzungen mit entzündungshemmender Wirkung.

Die entzündungshemmende Wirkung der erfindungsgemäß verwendeten 6,7-disubstituierten 2,2-Dialkylchromane oder -chromene manifestiert sich insbesondere in einer Hemmung der Ausschüttung des Entzündungsmediators Interleukin-1$\alpha$.

Die Substituenten $R^1$ und $R^2$ sind unabhängig voneinander ausgewählt aus einer OH-Gruppe, einer Methoxy-Gruppe und einer $CF_3CH_2O$-Gruppe. In einer bevorzugten Ausführungsform sind $R^1$ und $R^2$ unabhängig voneinander ausgewählt aus einer OH-Gruppe und einer Methoxy-Gruppe. In einer besonders bevorzugten Ausführungsform stellen $R^1$ eine OH-Gruppe und $R^2$ eine Methoxy-Gruppe dar.

Die Substituenten $R^3$ und $R^4$ stellen unabhängig voneinander eine $C_1$-$C_4$-Alkylgruppe dar. Unter $C_1$-$C_4$-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind $R^3$ und $R^4$ unabhängig voneinander ausgewählt aus einer Methyl-, Ethyl-, n-Propyl-, Isopropyl- und einer n-Butyl-Gruppe. Besonders bevorzugt stellen $R^3$ und $R^4$ unabhängig voneinander eine Methyl- oder Ethylgruppe dar. Außerordentlich bevorzugt ist, dass $R^3$ und $R^4$ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (I) oder (II) werden erfindungsgemäß in Mengen von 0,001 - 5 Gew.-%, bevorzugt 0,005 - 2 Gew.-% und besonders bevorzugt 0,01 - 1 Gew.-%, jeweils bezogen auf die gesamte Zusammensetzung, eingesetzt.

Die erfindungsgemäß hergestellten topischen kosmetischen oder pharmazeutischen Zusammensetzungen können sowohl als Leave-on-Produkt als auch als Rinse-off-Produkt formuliert werden. Geeignete Leave-on-Produkte sind Cremes, Salben, Pflegestifte, Make-ups, Lotionen, Masken, Packungen und Pflasterbeschichtungen, Sprays, Moussen sowie Deodorants und Antitranspirantien insbesondere zur Unterarmbehandlung, die in Form von O/W-Emulsionen, W/O-Emulsionen, O/W/O-Emulsionen, W/O/W-Emulsionen, O/W-Mikroemulsion W/O-Mikroemulsionen, wässrigen, alkoholischen oder wässrig-alkoholischen Gelen sowie als Öl vorliegen können. Geeignete Rinse-off-Produkte sind Waschgele, Waschcremes, Duschgele, Duschbäder, Seifen, Shampoos, Haarkonditioniermittel, Haarfärbezusammensetzungen, Haarwell- und Haarglättungsmittel sowie Tränkemulsionen und -lösungen für feste Substrate zur Reinigung und/oder Pflege der Haut und/oder der Haare.

In einer bevorzugten Ausführungsform enthalten die erfindungsgemäß hergestellten topischen kosmetischen oder pharmazeutischen Zusammensetzungen weiterhin mindestens einen Wirkstoff, ausgewählt aus anorganischen und organischen UV-Filtersubstanzen, Vitaminen, Provitaminen und Vitaminvorstufen der Gruppen A, B, C, E, H und K oder deren Derivaten, $\alpha$-Liponsäure, $\alpha$-Hydroxycarbonsäuren, Allantoin, $\alpha$-Bisabolol sowie Desoxyzuckern oder Desoxyzucker-Bausteine enthaltenden Polysacchariden.

Die erfindungsgemäß verwendeten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und - carbonsäureamiden, Ketotricyclo (5.2.1.0)decan, Benzalmalonsäureestem sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'-methoxyphenyl)propan-1,3-dion (Parsol® 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone (Uvasorb® HEB) sowie beliebige Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol® 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

In einer weiteren bevorzugten Ausführungsform enthalten die erfindungsgemäßen Zusammensetzungen mindestens ein Vitamin, Pro-Vitamin, eine Vitaminvorstufe oder deren Derivate. Hierbei sind solche Komponenten bevorzugt, die üblicherweise den Vitamingruppen A, B, C, E, H und K zugeordnet werden.
Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäß hergestellten Zubereitungen enthalten die Vitamin A-Komponente bevorzugt in Mengen von 0,05 - 1 Gew.-%, bezogen auf die gesamte Zusammensetzung.

Zur **Vitamin B-Gruppe** oder zu dem Vitamin B-Komplex gehören u. a.

- Vitamin $B_1$ (Thiamin)
- Vitamin $B_2$ (Riboflavin)
- Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das bevorzugt in Mengen von 0,05 bis 1 Gew.-%, bezogen auf die gesamte Zusammensetzung, enthalten ist.
- Vitamin $B_5$ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (III) eingesetzt werden.

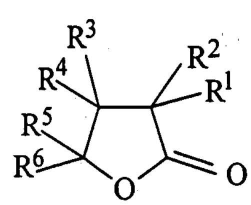

**(III)**

Bevorzugt sind die 2-Furanon-Derivate, in denen die Substituenten $R^1$ bis $R^6$ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten $C_2$-$C_4$ - Kohlenwasserstoffrest, einen gesättigten oder ein- oder zweifach ungesättigten, verzweigten oder linearen Mono-, Di- oder Trihydroxy-$C_2$-$C_4$-Kohlenwasserstoffrest oder einen gesättigten oder ein- oder zweifach ungesättigten, verzweigten oder linearen

Mono-, Di- oder Triamino-$C_2$-$C_4$ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) $R^1$ für eine Hydroxylgruppe, $R^2$ für ein Wasserstoffatom, $R^3$ und $R^4$ für eine Methylgruppe und $R^5$ und $R^6$ für ein Wasserstoffatom stehen. Das Stereoisomer (R)-Pantolacton entsteht beim Abbau von Pantothensäure.

Die genannten Verbindungen des Vitamin $B_5$-Typs sowie die 2-Furanonderivate sind bevorzugt in einer Gesamtmenge von 0,05 bis 10 Gew.-%, besonders bevorzugt 0,1 bis 5 Gew.-%, jeweils bezogen auf die gesamte Zusammensetzung, enthalten.

- Vitamin $B_6$ (Pyridoxin sowie Pyridoxamin und Pyridoxal).
- Vitamin $B_7$ (Biotin), auch als Vitamin H oder "Hautvitamin" bezeichnet. Biotin ist bevorzugt in Mengen von 0,0001 bis 1,0 Gew.-%, insbesondere 0,001 bis 0,01 Gew.-%, jeweils bezogen auf die gesamte Zusammensetzung, enthalten.

**Vitamin C** (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein. Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.

Unter **Vitamin F** werden üblicherweise essentielle Fettsäuren, insbesondere Linolsäure, Linolensäure und Arachidonsäure, verstanden.

**Vitamin H** ist eine andere Bezeichnung für Biotin oder Vitamin $B_7$ (siehe oben).

Zu den fettlöslichen Vitaminen der **Vitamin K**-Gruppe, denen das Grundgerüst des 2-Methyl-1,4-naphthochinons zugrunde liegt, gehören Phyllochinon (Vitamin $K_1$), Farnochinon oder Menachinon-7 (Vitamin K2) und Menadion (Vitamin $K_3$). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.

Bevorzugt enthalten die erfindungsgemäßen Mittel Vitamine, Provitamine und Vitaminvorstufen aus den Gruppen A, B, C, E und H oder deren Derivate. Vitamin A-palmitat (Retinylpalmitat), Panthenol und seine Derivate, Nicotinsäureamid, Ascorbylpalmitat, -acetat, Mg-Ascorbylphosphat, Na-Ascorbylphosphat, Natrium- und Magnesiumascorbat, die Tocopherolester, besonders Tocopherylacetat, und Biotin sind besonders bevorzugt.

In einer weiteren bevorzugten Ausführungsform enthalten die erfindungsgemäß verwendeten Zusammensetzungen Allantoin. Allantoin wird erfindungsgemäß in Mengen von 0,01 - 5 Gew.-%, bevorzugt 0,1 - 3 Gew.-% und besonders bevorzugt 0,5 - 1 Gew.-%, jeweils bezogen auf die gesamte Zusammensetzung, eingesetzt.

In einer weiteren bevorzugten Ausführungsform enthalten die erfindungsgemäß verwendeten Zusammensetzungen α-Bisabolol. α-Bisabolol wird erfindungsgemäß in Mengen von 0,01 - 5 Gew.-%, bevorzugt 0,1 - 3 Gew.-% und besonders bevorzugt 0,5 - 1 Gew.-%, jeweils bezogen auf die gesamte Zusammensetzung, eingesetzt.

In einer weiteren bevorzugten Ausführungsform enthalten die erfindungsgemäß verwendeten Zusammensetzungen α-Liponsäure. α-Liponsäure wird erfindungsgemäß in Mengen von 0,001 - 5 Gew.-%, bevorzugt 0,01 - 3 Gew.-% und besonders bevorzugt 0,1 - 1 Gew.-%, jeweils bezogen auf die gesamte Zusammensetzung, eingesetzt.

In einer weiteren bevorzugten Ausführungsform enthalten die erfindungsgemäß verwendeten Zusammensetzungen α-Hydroxycarbonsäuren. Die α-Hydroxycarbonsäuren sind erfindungsgemäß ausgewählt aus Glycolsäure, Milchsäure, Methylmilchsäure, 2-Hydroxybutansäure, 2-Hydroxypentansäure, 2-Hydroxyhexansäure, 2-Hydroxyheptansäure, 2-Hydroxyoctansäure, 2-Hydroxynonansäure, 2-Hydroxydecansäure, 2-Hydroxyundecansäure, 2-Hydroxydrodecansäure (α-Hydroxylaurinsäure), 2-Hydroxytetradecansäure (α-Hydroxymyristinsäure), 2-Hydroxyhexadecansäure (α-Hydroxypalmitinsäure), 2-Hydroxyoctadecansäure (α-Hydroxystearinsäure), 2-Hydroxyeicosansäure (α-Hydroxyarachidonsäure), Mandelsäure, Phenylmilchsäure, Glycerinsäure, 2,3,4-Trihydroxybutansäure mit den Isomeren Erythonsäure und Threonsäure, Ribonsäure, Arabinonsäure, Xylonsäure, Lyxonsäure, Allonsäure, Altronsäure, Gluconsäure, Mannonsäure, Gulonsäure, Idonsäure, Galactonsäure, Talonsäure, Glucoheptonsäure, Galactoheptonsäure, Tartronsäure, Äpfelsäure, Weinsäure, Zitronensäure, Schleimsäure (Galactarsäure), Glucarsäure sowie den physiologisch verträglichen Salzen der vorgenannten Säuren und ihren Lactonformen, insbesondere Gluconolacton, Galactolacton, Glucuronolacton, Galacturonolacton, Gulonolacton, Ribonolacton, Glucoheptonolacton, Mannonolacton, Galactoheptonolacton und Pantoyllacton.

Die α-Hydroxycarbonsäuren werden erfindungsgemäß in Mengen von 0,01 - 10 Gew.-%, bevorzugt 0,1 - 5 Gew.-% und besonders bevorzugt 1 - 3 Gew.-%, jeweils bezogen auf die gesamte Zusammensetzung, eingesetzt.

In einer weiteren bevorzugten Ausführungsform enthalten die erfindungsgemäß verwendeten Zusammensetzungen mindestens einen Desoxyzucker oder mindestens ein Desoxyzucker-Bausteine enthaltendes Polysaccharid. Als Desoxyzucker im Sinne der Erfindung werden vorzugsweise die L(-)-Fucose und die L(+)-Rhamnose verstanden. Fucose kommt z.B. als Baustein von Polysacchariden vor, die aus marinen Braunalgen (z. B. Fucus vesiculosus) isoliert werden können, Rhamnose stellt einen Polysaccharid-Baustein der Arabinsäure in Gummi arabicum dar. Entsprechende Handelsprodukte sind z. B. Fucogel 1000 (INCI-Bezeichnung Biosaccharide Gum-1) oder Rhamnosoft (INCI-Bezeichnung Biosaccharide Gum-2), beide von der Firma Solabia erhältlich.
Die Desoxyzucker oder Desoxyzucker-Bausteine enthaltenden Polysaccharide werden erfindungsgemäß in Mengen von 0,01 - 10 Gew.-%, bevorzugt 0,1 - 5 Gew.-% und besonders bevorzugt 1 - 3 Gew.-%, jeweils bezogen auf die gesamte Zusammensetzung, eingesetzt.

Die erfindungsgemäß verwendeten 6,7-disubstituierten 2,2-Dialkylchromane oder -chromene liegen als pulverförmiger Rohstoff vor und können unterhalb von ca. 40°C einfach in die vorgefertigten kosmetischen oder pharmazeutischen Zusammensetzungen eingearbeitet werden. Das vorherige Lösen der Chroman- oder Chromenderivate in einem geeigneten Lösemittel erhöht ihre Dispersibilität in der Formulierung. Geeignete Lösemittel sind die einwertigen $C_2$ - $C_4$-Alkohole, speziell Ethanol, Propanol, Isopropanol und Butanol, weiterhin Propylenglycol sowie Polyethylenglycole, speziell PEG-4, PEG-200 und PEG-400. Weiterhin geeignet sind polare Ölkomponenten, insbesondere die Triglyceride von gesättigten und ungesättigten $C_8$ - $C_{18}$-Fettsäuren. Bevorzugte Beispiele sind natürliche Triglyceride, z. B. Weizenkeimöl, sowie synthetische Triglyceride, z. B. das Handelsprodukt Myritol$^R$ 318. Schließlich lassen sich die erfindungsgemäß verwendeten Chroman- oder Chromen-Derivate mit Hilfe geeigneter Lösungsvermittler dispergieren. Geeignet sind insbesondere ethoxylierte Sorbitanfettsäuremonoester von $C_{12}$ - $C_{18}$-Fettsäuren, bevorzugt insbesondere Polysorbate-20 und Polysorbate-80.
Die erfindungsgemäß hergestellten Zusammensetzungen können weiterhin mindestens ein Polyol als Feuchthaltemittel enthalten. Bevorzugte Polyole sind Diole, Triole und höhere Alkohole, Polyglycerine, Polyethylenglycole sowie Mono- und Disaccharide. Unter den Diolen eignen sich $C_2$-$C_{12}$-Diole, insbesondere 1,2-Propylenglycol, Butylenglycole wie z. B. 1,2-Butylenglycol, 1,3-Butylenglycol und 1,4-Butylenglycol, Hexandiole, wie z. B. 1,6-Hexandiol. Weiterhin bevorzugt geeignet sind Glycerin und Polyglycerine, insbesondere Diglycerin und Triglycerin, 1,2,6-Hexantriol, sowie die Polyethylenglycole (PEG) PEG-400, PEG-600, PEG-1000, PEG-1550, PEG-3000 und PEG-4000.
Erfindungsgemäß geeignete Monosaccharide sind z. B. Glucose, Fructose, Galactose, Arabinose, Ribose und Mannose sowie Aminozucker wie z. B. Glucosamin oder Galactosamin.
Erfindungsgemäß geeignete Disaccharide sind aus zwei Monosaccharideinheiten zusammengesetzt, z. B. Saccharose oder Lactose. Ein besonders bevorzugtes Disaccharid ist Saccharose. Ebenfalls besonders bevorzugt ist die Verwendung von Honig, der überwiegend Glucose und Saccharose enthält.

Desweiteren lassen sich die erfindungsgemäß verwendeten Zusammensetzungen mit weiteren kosmetischen Wirkstoffen, z. B. Proteinhydrolysaten oder Pflanzenextrakten, formulieren. Viele dieser Substanzen zeigen eine Antifalten- oder Anti-Age-Wirkung, so dass entsprechende erfindungsgemäß verwendeten Zusammensetzungen auch als Antifalten- oder Anti-Age-Mittel verwendet werden können.

Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Kartoffel-, Mandel-, Erbsen- und Reisproteinhydrolysate. Entsprechende Handelsprodukte sind z. B. DiaMin® (Diamalt), Gluadin® (Cognis), Lexein® (Inolex) und Crotein® (Croda).
Wenngleich der Einsatz der Proteinhydrolysate als solche bevorzugt ist, können an ihrer Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon® (Cognis), Gluadin® (Cognis), Lexein® (Inolex), Crolastin® oder Crotein® (Croda).
Erfindungsgemäß einsetzbar sind auch kationisierte Proteinhydrolysate, wobei das zugrunde liegende Proteinhydrolysat vom Tier, von der Pflanze oder von marinen Lebensformen stammen oder biotechnologisch gewonnen sein kann. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate seien aufgeführt: Cocodimonium Hydroxypropyl Hydrolyzed Collagen, Cocodimonium Hydroxypropyl Hydrolyzed Casein, Steardimonium Hydroxypropyl Hydrolyzed Collagen.
Erfindungsgemäß sind die Proteinhydrolysate und deren Derivate in Mengen von 0,01 - 10 Gew.-% bezogen auf die gesamte Zusammensetzung, enthalten. Mengen von 0,1 bis 5 Gew.%, insbesondere 0,1 bis 3 Gew.-%, sind besonders

bevorzugt.

Die erfindungsgemäß hergestellten Zusammensetzungen können weiterhin Ölkomponenten enthalten. Geeignete Ölkomponenten sind ausgewählt aus den Estern von gesättigten und ungesättigten, linearen und verzweigten $C_3$-$C_{22}$-Fettsäuren und den Dimeren ungesättigter $C_{12}$-$C_{22}$-Fettsäuren (Dimerfettsäuren) mit a) einwertigen linearen, verzweigten und cyclischen $C_2$-$C_{18}$-Alkanolen, b) mehrwertigen linearen und verzweigten $C_2$-$C_6$-Alkanolen, c) Polyglycerinen der Formel $CH_2OH$-$CHOH$-$CH_2[$-$O$-$CH_2$-$CHOH$-$CH_2]_n$-$O$-$CH_2$-$CHOH$-$CH_2OH$ mit n = 0 - 8, und d) den Dicarbonsäureestern von linearen und verzweigten $C_2$-$C_{10}$-Alkanolen, den Benzoesäureestem von linearen und verzweigten $C_{10}$-$C_{20}$-Alkanolen, den $C_{12}$-$C_{22}$-Alkanolestern ein- und mehrwertiger $C_2$-$C_7$-Hydroxycarbonsäuren, die aromatisch sein können, sowie Mischungen dieser Komponenten.

Beispiele für erfindungsgemäß besonders geeignete Fettsäureester einwertiger linearer oder verzweigter $C_2$-$C_{18}$-Alkanole (Typ a) sind Octylethylhexanoat, Isopropylpalmitat, Hexyllaurat, Isopropylmyristat, ein Gemisch der Ester von Caprylsäure und Caprinsäure mit $C_{12}$-$C_{18}$-Fettalkoholen, das als Handelsprodukt Cetiol® LC (Cognis) erhältlich ist, $C_{12}$-$C_{15}$-Alkyloctanoat, Isodecylneopentanoat, Isononylisononanoat, 2-Ethylhexylpalmitat (z. B. Cegesoft® C 24 von Cognis), 2-Ethylhexylstearat, Cetearylisononanoat (Mischung aus Cetylisononanoat und Stearylisononanoat, z. B. Cetiol® SN (Cognis)), Cetearyloctanoat, Ethylstearat, Isopropylstearat, Butylstearat und Myristylpropionat. Weiterhin geeignet sind die Monoester des Isosorbid (1,4:3,6-Dianhydro-D-glucit) mit $C_2$-$C_{24}$-Fettsäuren, z. B. das Isosorbidmonolaurat.

Beispiele für erfindungsgemäß besonders geeignete $C_8$-$C_{22}$-Fettsäureester mehrwertiger $C_2$-$C_6$-Alkanole (Typ b) sind als pflanzliche und tierische Öle vorkommende Fettsäuretriglycerid-Öle, z. B. Sonnenblumenöl, Sojaöl, Sesamöl, Haselnußöl, Mandelöl, Olivenöl, sowie Distelöl (Safloröl). Besonders bevorzugt ist Distelöl. Erfindungsgemäß außerordentlich gut geeignet sind weiterhin synthetische Fettsäuretriglycerid-Öle. Besonders bevorzugt sind die Triglyceride von gesättigten $C_8$-$C_{10}$-Fettsäuren, wobei die Fettsäuren sowohl unverzweigt, wie z. B. bei den Handelsprodukten Myritol® 318 und Myritol® 331 (Cognis) oder Miglyol® 812 (Hüls), als auch verzweigt sein können, wie z. B. bei den Handelsprodukten Estol® GTEH 3609 (Uniqema) oder Myritol® GTEH (Cognis).

Weitere geeignete Ester dieses Typs sind die Fettsäureester des 1,2-Propylenglycols, des Neopentylglycols, z. B. Neopentylglycoldiheptanoat, des Trimethylolpropans und des Pentaerythrits, wobei die 1,2-Propylenglycolester bevorzugt sind. Beispiele für erfindungsgemäß bevorzugte Ölkomponenten sind die 1,2-Propylenglycoldiester von gesättigten, unverzweigten $C_8$-$C_{10}$-Fettsäuren, besonders bevorzugt die Handelsprodukte Myritol® PC und Miglyol® 840.

Beispiele für erfindungsgemäß besonders geeignete Fettsäureester von Polyglycerinen (Typ c) sind Polyglyceryl-2-dipolyhydroxystearat (Handelsprodukt Dehymuls® PGPH von Cognis), Polyglyceryl-3-diisostearat (Handelsprodukt Lameform® TGI von Cognis) und der Diester der Dilinolsäure mit Polyglyceryl-3-diisostearat (Handelsprodukt Isolan® PDI von Th. Goldschmidt).

Beispiele für erfindungsgemäß besonders geeignete Dicarbonsäureester (Typ d) sind Diisopropylsebacat, Diisopropyladipat, Dioctylmalat, Dioctylmaleat, Dicaprylylmaleat, Dioctyladipat, Di-n-butyladipat, Diisooctylsuccinat, Di-(2-hexyldecyl)-succinat und Diisotridecylacelaat.

Beispiele für erfindungsgemäß besonders geeignete Benzoesäureester von linearen oder verzweigten $C_{10}$-$C_{20}$-Alkanolen sind unter dem Warenzeichen Finsolv® (Hersteller: Finetex) erhältlich. Besonders bevorzugt sind die Handelsprodukte Finsolv® TN (Benzoesäure-C12-C15-alkylester), Finsolv® SB (Benzoesäureisostearylester) und Finsolv® BOD (Benzoesäureoctyldocecylester).

Als $C_8$-$C_{22}$-Fettalkoholester einwertiger oder mehrwertiger $C_2$-$C_7$-Hydroxycarbonsäuren sind die Ester der Glycolsäure, Milchsäure, Äpfelsäure, Weinsäure, Citronensäure und Salicylsäure erfindungsgemäß besonders geeignet. Solche Ester auf Basis von linearen $C_{14/15}$-Alkanolen und von in 2-Position verzweigten $C_{12/13}$-Alkanolen sind unter dem Warenzeichen Cosmacol® von der Fa. Nordmann, Rassmann GmbH & Co, Hamburg, zu beziehen. Besonders bevorzugt sind die Handelsprodukte Cosmacol® ESI, Cosmacol® EMI und Cosmacol® ETI.

Die Ölkomponenten sind in einer Menge von 1 - 20 Gew.-% in der erfindungsgemäßen Zusammensetzung enthalten. Bevorzugt ist ein Gehalt von 3 - 15 Gew.-%, besonders bevorzugt von 4 - 10 Gew.-% an Ölkomponenten, bezogen auf die Gesamtzusammensetzung.

Die erfindungsgemäß verwendeten Zusammensetzungen können weiterhin nichtionische Coemulgatoren enthalten. Bevorzugte Coemulgatoren sind lineare $C_{12}$-$C_{22}$-Fettalkohole und $C_{12}$-$C_{22}$-Fettsäurepartialester von $C_2$-$C_6$-Polyolen.

Geeignete Fettalkohole sind z.B. Cetylalkohol, Stearylalkohol, Oleylalkohol, Erucaalkohol, Ricinolalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.

Bevorzugte Polyol-Fettsäurepartialester sind Mono- und -diester von Glycerin oder 1,2-Propylenglycol mit gesättigten und ungesättigten linearen und verzweigten $C_{12}$-$C_{22}$-Fettsäuren sowie die Mono-, Sesqui- und -triester von Sorbitan mit gesättigten und ungesättigten linearen und verzweigten $C_{18}$-$C_{22}$-Fettsäuren. Besonders bevorzugt sind Glycerinmonostearat, Glycerinmonopalmitat, Glycerinmonolaurat, Glycerinmonooleat, Glycerindistearat, Glycerindipalmitat, Glycerindilaurat, Glycerindioleat sowie technische Gemische dieser Komponenten, weiterhin Sorbitanmonostearat,

Sorbitanmonooleat, Sorbitansesquioleat, Sorbitantristearat, Sorbitantrioleat sowie technische Gemische dieser Komponenten. Die Polyol-Fettsäurepartialester können ethoxyliert sein.

Weiterhin können außer den vorgenannten polaren Ölkomponenten und Coemulgatoren auch unpolare Ölkomponenten sowie Fettstoffe, die bei Raumtemperatur fest sind, enthalten sein. Zu diesen übrigen Öl- und Fettkomponenten zählen Fettsäuren sowie natürliche und synthetische Wachse, die sowohl in fester Form als auch flüssig oder in wäßriger Dispersion vorliegen können, sowie natürliche und synthetische unpolare kosmetische Öle.

Als Fettsäuren können eingesetzt werden lineare und/oder verzweigte, gesättigte und/oder ungesättigte $C_{8-30}$-Fettsäuren. Bevorzugt sind $C_{10-22}$-Fettsäuren. Beispiele sind die Isostearinsäuren und Isopalmitinsäuren. Weitere geeignete Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen.

Als natürliche oder synthetische Wachse können erfindungsgemäß eingesetzt werden feste Paraffine oder Isoparaffine, Pflanzenwachse wie Candelillawachs, Carnaubawachs, Espartograswachs, Fruchtwachse und Sonnenblumenwachs, Bienenwachse und andere Insektenwachse, Ozokerite, Ceresin, Walrat sowie Microwachse aus Polyethylen oder Polypropylen. Weiterhin geeignet sind die Triglyceride gesättigter und gegebenenfalls hydroxylierter $C_{16-30}$-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2 - 6 C-Atomen, Fettsäuremonoalkanolamide mit einem $C_{12-22}$-Acylrest und einem $C_{2-4}$-Alkanolrest, synthetische Fettsäure-Fettalkoholester, z. B. Stearylstearat oder Cetylpalmitat, Esterwachse aus natürlichen Fettsäuren und synthetischen $C_{20-40}$-Fettalkoholen (INCI-Bezeichnung C20-40 Alkyl Stearate) und Vollester aus Fettalkoholen und Di- und Tricarbonsäuren, z. B. Dicetylsuccinat oder Dicetyl-/stearyladipat, sowie Mischungen dieser Substanzen.

Vorteilhafte kosmetische Ölkörper, die erfindungsgemäß eingesetzt werden können, sind beispielsweise flüssige Paraffinöle, Isoparaffinöle und synthetische Kohlenwasserstoffe wie 1,3-Di-(2-ethyl-hexyl)-cyclohexan (Cetiol® S) sowie Di-n-alkylether mit insgesamt 12 bis 36, insbesondere 12 bis 24 C-Atomen, wie Di-n-octylether, Di-n-decylether, Di-nnonylether, Di-n-undecylether, n-Hexyl-n-octylether und n-Octyl-n-decylether. 1,3-Di-(2-ethyl-hexyl)-cyclohexan (Cetiol® S) und Di-n-octylether (Cetiol® OE) können bevorzugt sein. Weiterhin können symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC) eingesetzt werden.

Weitere erfindungsgemäß vorteilhaft einzusetzende Fettstoffe sind Siloxane. Die Siloxane können als Öle, Harze oder Gums vorliegen. Bevorzugte Siloxane sind Polydialkylsiloxane, wie z. B. Polydimethylsiloxan, Polyalkylarylsiloxane, wie z. B. Polyphenylmethylsiloxan, Polydialkylsiloxane, die Amin- und/oder Hydroxy-Gruppen enthalten sowie cyclische Silicone (INCI-Bezeichnung: Cyclomethicone), bevorzugt Octamethylcyclotetrasiloxan, Decamethylcyclopentasiloxan und Dodecamethylcyclohexasiloxan.

Weiterhin können die erfindungsgemäß verwendeten Zusammensetzungen mindestens ein Polymer enthalten. Die Polymere sind ausgewählt aus natürlichen und synthetischen anionischen, kationischen, nichtionischen und amphoteren Polymeren. Erfindungsgemäß bevorzugt sind synthetische und natürliche anionische und nichtionische Polymere.

Geeignete anionische Polymere enthalten Carboxylat- und/oder Sulfonatgruppen und als Monomere beispielsweise Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Innerhalb dieser Ausführungsform kann es bevorzugt sein, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionische Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 - 55 Mol-% Acrylamid und 30 - 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel® EG als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen

sind beispielsweise die Handelsprodukte Carbopol®. Weitere bevorzugte anionische Copolymere sind solche, die als Monomere 80 - 98 Gew.-% gewünschtenfalls substituierte Acrylsäure und 2 - 20 Gew.-% $C_{12}$-$C_{30}$-Fettalkoholmethacrylsäureester enthalten und vernetzt sein können. Solche Verbindungen sind z. B. die Handelsprbdukte Pemulen®.
Unter amphoteren Polymeren versteht man sowohl solche Polymere, die im Molekül sowohl freie Aminogruppen als auch freie -COOH- oder $SO_3$H-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen bzw. -COOHoder $SO_3$H-Gruppen enthalten. Ein Beispiel für ein erfindungsgemäß einsetzbares amphoteres Polymer ist das unter der Bezeichnung Amphome® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.
Die erfindungsgemäßen Mittel können weiterhin synthetische nichtionische Polymere enthalten. Geeignete nichtionische synthetische Polymere sind Polyvinylpyrrolidone und Vinylpyrrolidon/Vinylester-Copolymere, z. B. die Handelsprodukte Luviskol® (BASF), sowie Polyvinylalkohole.
Bei kationischen Polymeren unterscheidet man "temporär" und "permanent" kationische Polymere. Temporär kationische Polymere enthalten üblicherweise eine Aminogruppe, die bei bestimmten pH-Werten als quartäre Ammoniumgruppe vorliegt. Bevorzugt sind Chitosan und dessen Derivate, z. B. die Produkte Hydagen® CMF, Hydagen® HCMF, Kytamer® PC und Chitolam® NB/101. Besonders gut geeignete Chitosane weisen einen Deacetylierungsgrad von wenigstens 80 % und ein Molekulargewicht von $5 \cdot 10^5$ bis $5 \cdot 10^6$ g/mol auf. Zur Herstellung erfindungsgemäßer Zubereitungen muß das Chitosan in die Salzform überführt werden. Hierzu geeignete Säuren sind z. B. Essigsäure, Glycolsäure, Weinsäure, Apfelsäure, Citronensäure, Milchsäure, 2-Pyrrolidon-5-carbonsäure, Benzoesäure und Salicylsäure.
Weitere verwendbare kationische Polymere sind beispielsweise quaternisierte CelluloseDerivate, z. B. die Handelsprodukte Celquat® und Polymer JR®, insbesondere Celquat® H 100, Celquat® L 200 und Polymer JR®400, kationische Alkylpolyglycoside gemäß der DE-PS 44 13 686, kationisierter Honig, kationische Guar-Derivate, insbesondere die Produkte Cosmedia®Guar und Jaguar®, Polysiloxane mit quaternären Gruppen, wie z.B. die Handelsprodukte Q2-7224 (Dow Corning), Dow Corning® 929 Emulsion, SM-2059 (General Electric), SLM-55067 (Wacker) sowie Abil®-Quat 3270 und 3272 (Th. Goldschmidt), sowie die unter den Bezeichnungen Polyquaternium-2, Polyquatemium-17, Polyquaternium-18 und Polyquaternium-27 bekannten Polymeren mit quartären Stickstoffatomen in der Polymerhauptkette. Gleichfalls als kationische Polymere eingesetzt werden können die unter den Bezeichnungen Polyquaternium-24 bekannten Polymere.
Ebenfalls als kationisches Polymer verwendbar ist das, gewünschtenfalls vernetzte, Poly(methacryloyloxyethyltrimethylammoniumchlorid) mit der INCI-Bezeichnung Polyquaternium-37. Polyquaternium-37 wird häufig in Form einer nichtwässrigen Polymerdispersion eingesetzt. Solche Polymerdispersionen sind unter den Bezeichnungen Salcare® SC 95 und Salcare® SC 96 im Handel erhältlich.
Copolymere aus Methacryloyloxyethyltrimethylammoniumchlorid und nichtionischen Monomeren, bevorzugt Acrylamid, Methacrylamid, Acrylsäure-$C_{1-4}$-alkylester und Methacrylsäure-$C_{1-4}$-alkylester, die gegebenenfalls vemetzt sein können, sind im Handel unter dem Namen Salcare® SC 92 erhältlich.
Es ist erfindungsgemäß auch möglich, dass die verwendeten Zubereitungen mehrere, insbesondere zwei verschiedene Polymere gleicher Ladung und/oder jeweils ein ionisches und ein amphoteres und/oder ein synthetisches nichtionisches Polymer enthalten.

Weiterhin können die erfindungsgemäß verwendeteten Zusammensetzungen Pflanzenextrakte enthalten. Geeignete Pflanzenextrakte werden üblicherweise durch Extraktion der gesamten Pflanze, in einzelnen Fällen aber auch ausschließlich aus Blüten und/oder Blättern und/oder Samen und/oder anderen Pflanzenteilen, hergestellt. Erfindungsgemäß bevorzugt sind vor allem die Extrakte aus dem Meristem, also dem teilungsfähigen Bildungsgewebe der Pflanzen, und speziellen Pflanzen wie Hamamelis, Kamille, Ringelblume, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch (Althaea), Malve (Malva sylvestris), Veilchen, Blättern der Schwarzen Johannisbeere, Huflattich, Fünffingerkraut, Ginseng, Ingwerwurzel und Süßkartoffel.
Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden. Vorteilhaft eingesetzt werden können auch Extrakte aus Mikroorganismen, z. B. aus Hefen, bevorzugt aus Bäckerhefe.
Besonders bevorzugt sind die Extrakte aus Spirulina, Aloe Vera, Meristem, Hamamelis, Aprikose, Ringelblume, Guave, Süßkartoffel, Limette, Mango, Kiwi, Gurke, Malve, Eibisch und Veilchen. Die erfindungsgemäßen Mittel können auch Mischungen aus mehreren, insbesondere aus zwei, verschiedenen Pflanzenextrakten enthalten.
Sebumregulierende Wirkstoffe werden im Sinne der Erfindung besonders bevorzugt ausgewählt aus wasser- und öllöslichen Extrakten aus Hamamelis, Klettenwurzel und Brennnessel, Zimtbaumextract (z. B. Sepicontrol® A5 von der Firma Seppic), Chrysanthemenextrakt (z. B. Laricyl® von Laboratoires Sérobiologiques) und aus handelsüblichen Wirkstoffmischungen, z. B. Asebiol® BT 2 (INCI: Aqua, Hydrolyzed Yeast Protein, Pyridoxine, Niacinamide, Glycerin, Panthenol, Allantoin, Biotin) der Firma Laboratoires Sérobiologiques und Antifettfaktor® COS-218/2-A (von Cosmetochem, INCI: Aqua, Cetyl-PCA, PEG-8 Isolauryl Thioether, PCA, Cetyl Alcohol).

Weiterhin können die erfindungsgemäß verwendeten Zusammensetzungen zusätzliche Antioxidantien enthalten, die sowohl zur Stabilisierung der Fette gegen Autoxidation als auch als Radikalfänger zur vorbeugenden Hautpflege dienen. Besonders vorteilhaft werden die Antioxidantien ausgewählt aus Aminosäuren (z. B. Glycin, Histidin, Tyrosin, Tryptophan) und deren Derivaten, Imidazol und Imidazolderivaten (z. B. Urocaninsäure), Peptiden wie z. B. Carnosin und dessen Derivaten (z. B. Anserin), Carotinoiden, Carotinen (z. B. α-Carotin, β-Carotin, Lycopin) und deren Derivaten, weiteren Thioverbindungen, z. B. Thioglycerin, Thiosorbitol, Thioglycolsäure, Thioredoxin, Glutathion, Cystein, Cystin, Cystamin und deren Estern sowie deren Salzen, Dilaurylthiodipropionat, Distearylthiodipropionat, Thiodipropionsäure und deren Derivaten sowie Sulfoximinverbindungen (z. B. Buthioninsulfoximine, Homocysteinsulfoximin) in sehr geringen verträglichen Dosierungen (z. B. pmol/kg bis μmol/kg), ferner Metallchelatoren (z.B. EDTA, EGTA), Huminsäuren, Gallensäure, Gallenextrakten, Gallussäureestern (z. B. Propyl-, Octyl- und Dodecylgallat), Catechinen, ungesättigten Fettsäuren und deren Derivaten (z. B. γ-Linolensäure, Linolsäure, Arachidonsäure, Ölsäure), Folsäure und deren Derivaten, Hydrochinon und dessen Derivaten (z. B. Arbutin), Ubichinon und Ubichinol sowie deren Derivaten, Isoascorbinsäure und deren Derivaten, dem Coniferylbenzoat des Benzoeharzes, Rutin, Rutinsäure und deren Derivaten (z. B. Dinatriumrutinyldisulfat), Zimtsäure und deren Derivaten (z. B. Ferulasäure, Ethylferulat, Kaffeesäure), Kojisäure, Butylhydroxytoluol, Butylhydroxyanisol, Nordihydroguajakharzsäure, Nordihydroguajaretsäure, Trihydroxybutyrophenon, Harnsäure und deren Derivaten, Selen und Selen-Derivaten (z. B. Selenmethionin) sowie Stilbenen und Stilben-Derivaten. Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden.
Die Gesamtmenge der zusätzlichen Antioxidantien, das heißt ohne die erfindungsgemäß verwendeten 6,7-disubstituierten 2,2-Dialkyl-Chromane oder -chromene, beträgt erfindungsgemäß 0,001 - 10 Gew.-%, vorzugsweise 0,05 - 5 Gew.-% und insbesondere 0,1 - 2 Gew.-%, bezogen auf das Gesamtgewicht der Zusammensetzungen.

Besonders bevorzugte Komponenten mit kosmetischer und dermatologischer Wirkung sind außerdem Pyrrolidoncarbonsäure sowie Sterine, z. B. Ergosterin, Stigmasterin, Sitosterin und Mykosterine.
Als weitere Hilfsmittel können die erfindungsgemäßen Zusammensetzungen übliche galenische Hilfsstoffe in den gebräuchlichen Mengen enthalten. Solche Hilfsstoffe sind z.B. Verdickungsmittel für die wässrige Phase, z.B. vom Typ der Schichtsilikate oder der pyrogenen Kieselsäuren. Weitere Hilfsstoffe sind Konservierungsstoffe wie z. B. Phenoxyethanol, p-Hydroxybezoesäureester, Sorbinsäure, Komplexbildner (z.B. Trilon® B) sowie Farb- und Duftstoffe.

Weiterhin können die erfindungsgemäß verwendeten Zusammensetzungen Antitranspirant-Wirkstoffe enthalten. Als Antitranspirant-Wirkstoffe eignen sich wasserlösliche adstringierende oder einweißkoagulierende metallische Salze, insbesondere anorganische und organische Salze des Aluminiums, Zirkoniums und Zinks sowie beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Geeignet sind beispielsweise Aluminiumlactat, Natrium-Aluminium-Chlorhydroxylactat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat, Aluminium-Zirkonium-Chlorohydrat-Glycin-Komplexe und Komplexe von basischen Aluminiumchloriden mit Propylenglycol oder Polyethylenglycol. Bevorzugt enthalten die flüssigen Wirkstoffzubereitungen ein adstringierendes Aluminiumsalz, insbesondere Aluminiumchlorohydrat, und/ oder eine Aluminium-Zirkonium-Verbindung. Sie sind in den erfindungsgemäß hergestellten Zusammensetzungen in einer Menge von 1 - 40 Gew.-%, vorzugsweise 5 - 30 Gew.-% und insbesondere 10 - 25 Gew.-% enthalten (bezogen auf die Menge der Aktivsubstanz in der gesamten Zusammensetzung).

Ebenfalls als kosmetische oder dermatologische Wirkstoffe erfindungsgemäß geeignet sind Deodorantien. Als Deodorantien können Duftstoffe, antimikrobielle, antibakterielle oder keimhemmende Stoffe, enzymhemmende Stoffe, Antioxidantien oder Geruchsadsorbentien (z. B. Zinkricinoleat) eingesetzt werden.
Geeignete antimikrobielle, antibakterielle oder keimhemmende Stoffe sind insbesondere Organohalogenverbindungen sowie -halogenide, quartäre Ammoniumverbindungen, eine Reihe von Pflanzenextrakten und Zinkverbindungen.

Weiterhin können Emulgatoren enthalten sein. Bevorzugte Emulgatoren sind ausgewählt aus der Gruppe der nichtionischen Ethylenoxid-Addukte, oder einer Kombination aus einem hydrophilen, nichtionischen Emulgator mit

einem HLB-Wert von bevorzugt 10-19 und einem lipophilen Coemulgator. Unter dem HLB-Wert soll dabei eine Größe verstanden werden, die aus der Struktur des Emulgators errechnet werden kann gemäß

$$HLB = \frac{100 - L}{5}$$

worin L der Gewichtsanteil (in %) der lipophilen Gruppen, z. B. der Fettalkyl- bzw. Fettacylgruppen im Emulgator ist.

Erfindungsgemäß verwendbare hydrophile nichtionische Emulgatoren sind beispielsweise

- Anlagerungsprodukte von 4 bis 30 Mol Ethylenoxid und/oder 0 bis 5 Mol Propylenoxid an lineare $C_8$-$C_{30}$-Fettalkohole, an $C_{12}$-$C_{22}$-Fettsäuren und an $C_8$-$C_{15}$-Alkylphenole,
- $C_{12}$-$C_{22}$-Fettsäuremono- und -diester von Anlagerungsprodukten von 1 bis 30 Mol Ethylenoxid an $C_3$-$C_6$-Polyole, insbesondere an Glycerin,
- Ethylenoxid- und Polyglycerin-Anlagerungsprodukte an Methylglucosid-Fettsäureester, Fettsäurealkanolamide und Fettsäureglucamide,
- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, z. B. das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten $C_8$-$C_{22}$-Fettsäuren,
- Sterole (Sterine),
- Phospholipide,
- Fettsäureester von Zuckern und Zuckeralkoholen, wie Sorbit,
- Polyglycerine und Polyglycerinderivate, beispielsweise Polyglycerinpoly-12-hydroxystearat (Handelsprodukt Dehymuls® PGPH).

Bevorzugte hydrophile Emulgatoren sind die Ethylenoxid-Anlagerungsprodukte an lineare $C_{16}$-$C_{30}$-Fettalkohole oder an Partialester von $C_3$-$C_6$-Polyolen mit $C_{12}$-$C_{22}$-Fettsäuren. Es kann besonders bevorzugt sein, als hydrophile Emulgatoren Alkylpolyglycoside der Formel $RO - (Z)_x$ einzusetzen, in der R eine $C_8$-$C_{22}$-Alkyl- oder Alkenylgruppe, Z einen Monosaccharidrest, insbesondere Glucose, und dessen Oligomerisationsgrad x eine Zahl von 1,1 bis 5, insbesondere von 1,2 bis 1,4 darstellt.

Die Emulgatoren sind in einer Menge von 0,2 - 10 Gew.-%, bevorzugt 0,5 - 8 Gew.-% und besonders bevorzugt 1 - 7 Gew.-%, jeweils bezogen auf die gesamte Zusammensetzung, enthalten.

Ein weiterer Gegenstand der Erfindung sind topische kosmetische oder pharmazeutische Zusammensetzungen mit entzündungshemmender Wirkung mit einem Gehalt an 6,7-disubstituierten 2,2-Dialkylchromanen oder -chromenen der allgemeinen Formeln (I) oder (II),

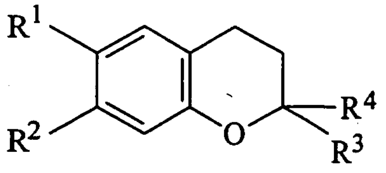

(I)

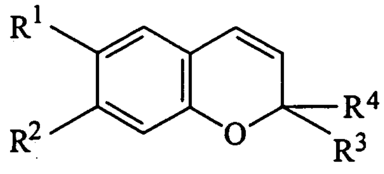

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen,
gekennzeichnet durch einen Gehalt an einem weiteren Wirkstoff, ausgewählt aus :

- den Vitaminen, Provitaminen und Vitaminvorstufen der Gruppen A, B, C, E, H und K oder deren Derivaten,
- α-Bisabolol,
- α-Liponsäure,
- α-Hydroxycarbonsäuren sowie

- Desoxyzuckern oder Desoxyzucker-Bausteine enthaltenden Polysacchariden.

Die folgenden Beispiele sollen den Gegenstand der Erfindung näher erläutern, ohne ihn hierauf zu beschränken.

**Beispiele**

1. Untersuchungen an Mehrschicht-Hautmodellen

Die Wirkung der erfindungsgemäß verwendeten 6,7-disubstituierten 2,2-Dialkylchromane oder -chromene wurde an einem mehrschichtigen in-vitro-Hautmodell untersucht. Das Hautmodell ist ein humanes Hautäquivalent, das aus einer Dermis mit Fibroblasten und einer Epidermis aus Keratinozyten besteht.

Diese Mehrschicht-Struktur entsteht in einem speziellen Kultivierungsverfahren. Es wurden zunächst dermale Äquivalente (DE) produziert, indem eine Suspension von 2 x $10^5/cm^2$ Fibroblasten aus humaner Vorhaut in einem Kulturmedium auf eine aus Chitosan, Collagen und Glycosaminoglycanen bestehende Matrix aufpipettiert wurde (Matrix beschrieben bei Collombel, C. et al.: Biomaterials with a base of collagen, chitosane and glycosaminoglycans, process for preparing them and their application in human medicine, US Patent 5166187). Das Kulturmedium bestand aus Dulbecco's Modified Eagle's Medium (DMEM), supplementiert mit 10% fetalem Kälberserum (FCS), 25 µg/ml Gentamycin, 100 UI/ml Penicillin, 1 µg/ml Amphotericin B, 50 µg/ml Natriumascorbat und 4 mM L-Glutamin. Die dermalen Äquivalente wurden 14 Tage in diesem Medium bei 37°C in einer Atmosphäre $CO_2$/Luft (5%/95%, v/v) und 90% Luftfeuchtigkeit inkubiert, wobei das Medium jeden Tag erneuert wurde. Für die Hautäquivalente (SE) wurden Keratinozyten aus humaner Vorhaut in einer Dichte von 200.000 Zellen/$cm^2$ auf die 14 Tage alten DEs ausgesät und unter submersen Bedingungen in einem Medium, bestehend aus 60 % DMEM, 30 % HAM F12 und 10% FCS, supplementiert mit 25 µg/ml Gentamycin, 100 UI/ml Penicillin, 1 µg/ml Amphotericin B, 50 µg/ml Natriumascorbat, 4 mM L-Glutamin, 10 ng/ml Epidermalem Wachstumsfaktor (EGF), 0,4µg/ml Hydrocortison, 0,12UI/ml Insulin, $10^{-9}$ M Choleratoxin, 5 ng/ml Transferrin und 180 µM Adenin, für weitere 7 Tage inkubiert. Die Hautäquivalente wurden dann an der Luft-Flüssigkeitsgrenze (Air-Liquid-Interface) für weitere 14 Tage in modifiziertem Keratinozytenmedium (DMEM-HAM F12, supplementiert mit 0,4 µg/ml Hydrocortison und 0,12 UI/ml Insulin) kultiviert.

Im Vergleich zu üblicherweise verwendeten Monolayer-Kulturen entspricht dieses Ganzhautmodell sehr viel besser der in-vivo-Situation, da Keratinozyten und Fibroblasten in engem Kontakt zueinander stehen und, wie in vivo, Signalstoffe austauschen können.

2. Behandlung der Ganzhautmodelle:

Zur Durchführung der Prüfungen wurde eine Cremeformulierung auf Basis der nachstehend aufgeführten Versuchsrezeptur mit 0,01 Gew.-% 3,4-Dihydro-7-methoxy-2,2-dimethyl-2H-1-benzopyran-6-ol (Lipochroman-6 von Lipotec) hergestellt, wobei die Differenz zu 100 Gew.-% mit Wasser ausgeglichen wurde. Anschließend wurde der Effekt der Lipochroman-6-haltigen Creme auf das Ganzhautmodell im Unterschied zu einer Vergleichscreme (gleiche Cremeformulierung mit 0,05 Gew.-% Propylgallat als Antioxidans an Stelle von Lipochroman-6) bewertet. Dazu wurden die Hautmodelle topisch jeweils dreimal im Abstand von 12 Stunden mit 5 µl der verschiedenen Cremeformulierungen behandelt. Nach den ersten beiden Creme-Applikationen wurden die Hautmodelle 12 Stunden inkubiert. Nach der dritten Applikation wurden sie 1 Stunde lang inkubiert und anschließend mit simuliertem Sonnenlicht bestrahlt.

| Versuchsrezepturen | | |
|---|---|---|
| **Komponente** | **Erfindungsgemäße Creme Menge (Gew.-%)** | **Vergleichscreme Menge (Gew.-%)** |
| Montanov®202 | 3,0 | 3,0 |
| Isopropylstearat | 3,0 | 3,0 |
| Myritol® 331 | 3,0 | 3,0 |
| Paraffinöl | 1,0 | 1,0 |
| Cegesoft® C24 | 1,0 | 1,0 |
| Lanette® 22 | 1,0 | 1,0 |
| Cutina® MDV | 1,0 | 1,0 |

(fortgesetzt)

| Versuchsrezepturen | | |
|---|---|---|
| **Komponente** | **Erfindungsgemäße Creme Menge (Gew.-%)** | **Vergleichscreme Menge (Gew.-%)** |
| Vitamin E-acetat | 0,5 | 0,5 |
| Parsol® 1789 | 1,0 | 1,0 |
| Eusolex® 6300 | 2,0 | 2,0 |
| Uvinul® T 150 | 1,25 | 1,25 |
| Baysilon® M 350 | 0,5 | 0,5 |
| Tego Carbomer® 140 2%ig | 25 | 25 |
| 1,2-Propylenglykol | 7,0 | 7,0 |
| NaOH 10%ig | ad pH 4,8-5,2 | ad pH 4,8-5,2 |
| Lipochroman-6 | 0,01 | - |
| Propylgallat | - | 0,05 |
| Wasser demineralisiert | ad 100 | ad 100 |

3. Nachweis der Hemmung der Ausschüttung des Entzündungsmediators Interleukin-1α durch 6,7-disubstituierte 2,2-Dialkylchromane oder-chromene:

Die, wie unter Punkt 2 beschrieben, vorbehandelten Hautmodelle wurden mit simuliertem Sonnenlicht bestrahlt. Zur Kontrolle wurden auch unbehandelte Hautmodelle bestrahlt. Für die Bestrahlung wurde ein Sonnenlichtsimulator SOL 500 der Firma Hönle mit dem Filter H2 (295-400 nm) verwendet. Da das simulierte Sonnenlicht nur einen sehr geringen UV-B-Anteil aufweist, wurde die Strahlendosis nur für den UV-A-Anteil gemessen. Die einzelnen Hautmodelle wurden mit unterschiedlichen UV-A-Dosen bestrahlt. Anschließend wurde das gebildete Interleukin-1α präpariert und mit einem handelsüblichen Assay bestimmt. Die Ergebnisse sind in Tabelle 1 zusammengestellt.

Tabelle 1:

| Interleukin-1α-Ausschüttung von Epidermis-Hautkulturen nach Bestrahlung mit simuliertem Sonnenlicht [pg Interleukin-1α] | | | |
|---|---|---|---|
| **UV-A-Strahlungsdosis [J/cm$^2$]** | **unbehandeltes Hautmodell** | **Hautmodell, mit Vergleichscreme vorbehandelt** | **Hautmodell, mit erfindungsgemäßer Creme vorbehandelt** |
| 0 | 15 | 15 | 15 |
| 6 | 15 | 14 | 15 |
| 9 | 15 | 16 | 15 |
| 12 | 20 | 18 | 15 |
| 15 | 29 | 18 | 15 |
| 18 | 41 | 23 | 18 |
| 21 | 96 | 75 | 17 |

4. Vitalitätsbestimmung mit Hilfe des MTT-Tests

Die, wie unter Punkt 2 beschrieben, vorbehandelten Hautmodelle wurden mit simuliertem Sonnenlicht bestrahlt. Zur Kontrolle wurden auch unbehandelte Hautmodelle bestrahlt. Für die Bestrahlung wurde ein Sonnenlichtsimulator SOL 500 der Firma Hönle mit dem Filter H2 (295-400 nm) verwendet. Da das simulierte Sonnenlicht nur einen sehr geringen UV-B-Anteil aufweist, wurde die Strahlendosis nur für den UV-A-Anteil gemessen. Die einzelnen Hautmodelle wurden mit unterschiedlichen UV-A-Dosen bestrahlt. Anschließend wurde die Vitalität der Zellen mit Hilfe

des MTT-Tests bestimmt.

Der MTT-Test liefert Informationen über die Zellproliferation und Zytotoxizität. Im Test wird die metabolische Aktivität lebender Zellen bestimmt. Das Tetrazoliumsalz 3-[4,5-Dimethylthiazol-2-yl]-2,5-diphenyltetrazoliumbromid (MTT) wird in lebenden Zellen reduziert und in ein wasserunlösliches Formazansalz umgewandelt. Das Formazansalz wird extrahiert und photometrisch quantifiziert. Die Menge an gebildetem Formazansalz ist ein Maß für die Anzahl lebender Zellen in der untersuchten Probe. Die exakte Duchführung des Tests ist in J. Immunol. Methods 65, 55, 1983 (T. Mosmann) offenbart, worauf hier explizit Bezug genommen wird.

Zur Herstellung der MTT-Lösung wurden 2 ml einer MTT-Lösung (Konz. 1 mg MTT/ml in phosphate buffered saline = PBS) in jedes Well einer 24-Well-Schale pipettiert. Die gemäß Punkt 2 vorbehandelten und bestrahlten Hautmodelle wurden in die Schale überführt und 3 Stunden lang bei 37°C in einer Atmosphäre $CO_2$/Luft (5%/95%, v/v) und 90 % Luftfeuchtigkeit inkubiert. Nach beendeter Inkubation wurden die Hautmodelle in Zentrifugenröhrchen überführt und das gebildete Formazansalz mit je 4 ml Extraktionsmittel (292 ml Isopropanol + 8 ml 1M HCl) 1,5 Stunden auf dem Schüttler extrahiert. Die optische Dichte eines Aliquots von 200 µl wurde in einer 96-Well-Platte bei einer Wellenlänge von 540 nm gemessen (Titertek Multiscan MCC 340, Fa. Flow Laboratories).
Die Ergebnisse sind in Tabelle 2 zusammengestellt.

Tabelle 2:

| Vitalität von Epidermis-Hautkulturen nach Bestrahlung mit simuliertem Sonnenlicht [%, bezogen auf unbehandeltes nicht-bestrahltes Hautmodell] | | | |
|---|---|---|---|
| **UV-A-Strahlungsdosis [J/cm$^2$]** | **unbehandeltes Hautmodell** | **Hautmodell, mit Vergleichscreme vorbehandelt** | **Hautmodell, mit erfindungsgemäßer Creme vorbehandelt** |
| 0 | 100 | 128 | 155 |
| 6 | 115 | 120 | 132 |
| 9 | 120 | 122 | 130 |
| 12 | 105 | 105 | 120 |
| 15 | 100 | 110 | 128 |
| 18 | 80 | 100 | 116 |
| 21 | 62 | 100 | 142 |

| **5. Erfindungsgemäße Beispielrezepturen (Mengenangaben in Gewichtsteilen)** | | | | |
|---|---|---|---|---|
| | | **5.1** | **5.2** | **5.3** |
| Phase 1 | Distelöl | 3,0 | 3,0 | 3,0 |
| | Myritol® PC | 3,5 | 3,5 | 3,5 |
| | Lanette® 22 | 3,0 | 3,0 | 3,0 |
| | Cutina® GMS-V | 3,0 | 3,0 | 3,0 |
| | Stenol® 16/18 | 2,0 | 2,0 | 2,0 |
| | Isopropylstearat | 6,0 | 6,0 | 6,0 |
| | Baysilon® M350 | 1,0 | 1,0 | 1,0 |
| | Controx® KS | 0,05 | 0,05 | 0,05 |
| | Parsol® 1789 | 2,0 | 2,0 | - |
| | Parsol® MCX | 3,0 | 3,0 | - |
| | Eusolex® 4360 | - | - | 0,5 |
| | Eusolex® 6300 | 3,0 | 3,0 | 2,0 |
| | Uvinul® T 150 | 2,5 | 2,5 | - |

(fortgesetzt)

| 5. Erfindungsgemäße Beispielrezepturen (Mengenangaben in Gewichtsteilen) | | | | |
|---|---|---|---|---|
| | | **5.1** | **5.2** | **5.3** |
| | Propylparaben | 0,2 | 0,2 | 0,2 |
| Phase 2 | Glycerin | 5,0 | 5,0 | 5,0 |
| | Methylparaben | 0,2 | 0,2 | 0,2 |
| | Wasser | 54,95 | 48,95 | 58,95 |
| Phase 3 | V-Protein flüssig (COS 152/22-A) | - | 9,0 | 9,0 |
| | Hibiscin® HP-LS-9198 | 3,0 | - | - |
| Phase 4 | Granlux® MSN 50 | 2,5 | 2,5 | - |
| Phase 5 | Citronensäure | 0,1 | 0,1 | 0,1 |
| Phase 6 | Lipochroman-6 | 0,01 | 0,01 | 0,01 |
| | Retinol | 0,1 | - | - |
| | Na-Ascorbyl-phosphat | - | 0,1 | - |
| Phase 7 | Sepigel® 305 | 2,0 | 2,0 | 2,0 |

Herstellung:

Fett- und Wasserphase (Phase 1 und Phase 2) wurden getrennt voneinander auf etwa 80° C erhitzt. Die Wasserphase wurde unter dem Homogenisator vorgelegt. Dann wurden Phase 3 und Phase 4 zugegeben. Anschließend wurde etwa 1 Minute lang homogenisiert. Dann wurde die Fettphase hinzugegeben und erneut etwa 1 Minute lang homogenisiert. Nach dem Abkühlen auf etwa 30° C wurden Lipochroman-6 und die Vitamin-Wirkstoffe zugegeben und der pH-Wert mit Citronensäure auf einen Wert im Bereich von 4,8 - 5,2 eingestellt. Anschließend wurde das Polymer (Sepigel® 305) hinzugegeben. Es bildete sich eine stabile Emulsion, nach dem Abkühlen auf 20° C eine glatte Creme von hoher Stabilität.

| **Komponente** | **5.4** | **5.5** | **5.6** | **5.7** |
|---|---|---|---|---|
| Hostaphat® KL 340 N | 1,5 | 1,5 | 1,5 | 1,5 |
| Ethylhexylpalmitat | 3,0 | 3,0 | 3,0 | 3,0 |
| Paraffinöl | 8,0 | 8,0 | 8,0 | 8,0 |
| Tocopherylacetat | 0,01 | 0,01 | 0,01 | 0,01 |
| Lipochroman-6® | 0,01 | 0,01 | 0,01 | 0,01 |
| Carbomer | 0,2 | 0,2 | 0,2 | 0,05 |
| NaOH | ad pH 4,8 - 5,2 | ad pH 4,8 - 5,2 | ad pH 4,8 - 5,2 | ad pH 4,8 - 5,2 |
| Propylenglycol | 10,0 | 10,0 | 10,0 | 10,0 |
| Methylparaben | 0,5 | 0,5 | 0,5 | 0,5 |
| Propylparaben | 0,5 | 0,5 | 0,5 | 0,5 |
| α-Bisabolol | 0,5 | - | - | - |
| α-Liponsäure | - | 0,05 | - | - |
| Citronensäure | 0,5 | - | - | - |
| Milchsäure | - | 0,5 | - | - |
| Gluconolacton | - | - | 0,5 | - |
| Fucogel® 1000 | - | - | - | 0,3 |

(fortgesetzt)

| Komponente | 5.4 | 5.5 | 5.6 | 5.7 |
|---|---|---|---|---|
| Wasser | ad 100 | ad 100 | ad 100 | ad 100 |

Herstellung:

Fett- und Wasserphase wurden getrennt voneinander auf etwa 80° C erhitzt. Die Wasserphase wurde unter dem Homogenisator vorgelegt. Dann wurde die Fettphase hinzugegeben und etwa 1 Minute lang homogenisiert. Nach dem Abkühlen auf etwa 30° C wurden Lipochroman-6 und die übrigen kosmetischen Wirkstoffe zugegeben und der pH-Wert mit Citronensäure auf einen Wert im Bereich von 4,8 - 5,2 eingestellt. Anschließend wurde das Carbomer hinzugegeben.

| Komponente | 6.1 |
|---|---|
| Isopropylpalmitat | 4,0 |
| Paraffinöl | 10,0 |
| Pluronic® L 44 | 2,0 |
| Cutina® GMS-V | 1,0 |
| Glycerin 99,5% DAB | 1,0 |
| Pyrrolidoncarbonsäure-natriumsalz | 0,5 |
| Phenonip® | 0,4 |
| Euxyl® K 400 | 0,1 |
| Kaliumsorbat | 0,3 |
| Tocopherylacetat | 0,5 |
| Lipochroman-6® | 0,01 |
| Citronensäure | 0,12 |
| Wasser | ad 100 |

Mit den erhaltenen Emulsionen 5.4 bis 5.7 und 6.1 wurden Vliestücher aus einer PolyesterNiskose-Mischung getränkt. Die Tücher aus Beispiel 5.7 wurden anschließend bis auf einen Wassergehalt von weniger als 10 Gew.-% getrocknet.
Die so erhaltenen Vliestücher können zur Reinigung, insbesondere auch zum Abschminken und zur Pflege der Haut, insbesondere auch im empfindlichen Augenbereich, verwendet werden. Die Tücher aus den Beispielen 5.4 bis 5.6 und 6.1 können direkt eingesetzt werden, die Tücher aus Beispiel 5.7 können vor der Anwendung mit Wasser befeuchtet werden.

| Liste der verwendeten Inhaltsstoffe | | | |
|---|---|---|---|
| **Komponente** | **INCI-Bezeichung** | | **Hersteller** |
| Baysilonöl® M 350 | Dimethicone | Polydimethylsiloxan | GE-Bayer-Silicones |
| Controx® KS | Tocopherol, Hydrogenated Palm Glycerides Citrate | Tocopherol-Mono-diglyceride-citrat-Gemisch | Cognis |
| Cutina® GMS-V | Glyceryl Stearate | Glycerin-mono-di-palmitat-stearat, pflanzlich | Cognis |
| Eusolex® 4360 | Benzophenone-3 | | Merck |
| Eusolex® 6300 | 4-Methylbenzylidene Camphor | | Merck |

(fortgesetzt)

| Liste der verwendeten Inhaltsstoffe | | | |
|---|---|---|---|
| **Komponente** | **INCI-Bezeichung** | | **Hersteller** |
| Euxyl® K 400 | Methyldibromo Glutaronitrile, Phenoxyethanol | | Schülke & Mayr |
| Fucogel® 1000 | Biosaccharide Gum-1 | | Solabia |
| Granlux® MSN 50 | Titanium dioxide, Apricot Kernel Oil PEG-40 Esters, Cetearyl Glucoside, Polydecene | Dispersion mit 45 - 55 Gew.-% $TiO_2$, Partikelgröße 22 nm | Oy Granula AB, Finnland |
| Hibiscin® HP-LS-9198 | Water, Hibiscus esculentus Seed Extract, Phenoxyethanol | | Laboratoires Sérobiologique s |
| Hostaphat® KL 340 N | Trilaureth-4 Phosphate | Phosphorsäuretris ($C_{12-14}$-Alkohol+4-EO) ester | Clariant |
| Kessco® IPS | Isopropyl Stearate | | Akzo Nobel |
| Lanette® 22 | Behenyl Alcohol | | Cognis |
| Lipochroman®-6 | Dimethylmethoxy Chromanol | 4-Dihydro-7-methoxy-2,2-dimethyl-2H-1-benzopyran-6-ol | Lipotec S.A: |
| Myritol® PC | Propylene Glycol Dicaprylate/Dicaprate | | Cognis |
| Parsol® 1789 | Butyl Methoxydibenzoylmethane | | Roche-Givaudan |
| Parsol® MCX | Octyl Methoxycinnamate | | Roche-Givaudan |
| Phenonip® | PHENOXYETHANOL, METHYL-PARABEN, ETHYLPARABEN, PROPYLPARABEN, BUTYL-PARABEN | | Nipa |
| Pluronic® L 44 | Poloxamer 124 | | BASF |
| Sepigel® 305 | Polyacrylamide, C13-14 Isoparaffin, Laureth-7 | | SEPPIC |
| Stenol® 16/18 | Cetearyl Alcohol | Cetyl-/Stearylalkohol (1 : 2) | Cognis |
| Uvinul® T 150 | Octyl Triazone | | BASF |
| V-Protein flüssig (COS 152/22-A) | Water, Propylene Glycol, Hydrolyzed Pea Protein (Pisum sativum) | | Cosmetochem |

**Patentansprüche**

1. Verwendung von 6,7-disubstituierten 2,2-Dialkylchromanen oder -chromenen der allgemeinen Formeln (I) oder (II),

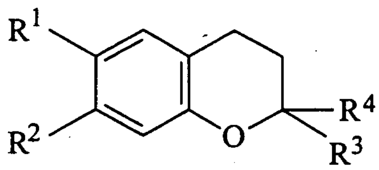

(I)

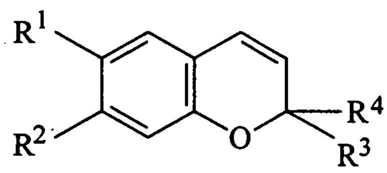

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, zur Herstellung topischer kosmetischer oder pharmazeutischer Zusammensetzungen mit entzündungshemmender Wirkung.

2. Verwendung gemäß Anspruch 1, **dadurch gekennzeichnet, dass** die Ausschüttung von Interleukin-1$\alpha$ gehemmt wird.

3. Verwendung gemäß Anspruch 1 oder 2, **dadurch gekennzeichnet, dass** $R^1$ und $R^2$ unabhängig voneinander eine OH-Gruppe oder eine Methoxy-Gruppe darstellen.

4. Verwendung gemäß einem der Ansprüche 1 bis 3, **dadurch gekennzeichnet, dass** $R^3$ und $R^4$ unabhängig voneinander ausgewählt sind aus einer Methyl-, Ethyl-, n-Propyl-, Isopropyl- und n-Butylgruppe.

5. Verwendung gemäß einem der Ansprüche 1 bis 4, **dadurch gekennzeichnet, dass** $R^1$ eine OH-Gruppe, $R^2$ eine Methoxy-Gruppe und $R^3$ und $R^4$ eine Methylgruppe darstellen.

6. Verwendung gemäß einem der Ansprüche 1 bis 5, **dadurch gekennzeichnet, dass** die 6,7-disubstituierten 2,2-Dialkylchromane oder -chromene der allgemeinen Formeln (I) oder (II) in Mengen von 0,001 - 5 Gew.-%, bezogen auf die gesamte Zusammensetzung, eingesetzt werden.

7. Verwendung gemäß einem der Ansprüche 1 bis 6, **dadurch gekennzeichnet, dass** die topischen kosmetischen oder pharmazeutischen Zusammensetzungen ausgewählt sind aus Leave-on-Produkten wie Cremes, Salben, Pflegestiften, Make-ups, Lotionen, Masken, Packungen und Pflasterbeschichtungen, Sprays, Moussen, Deodorants und Antitranspirantien insbesondere zur Unterarmbehandlung, die in Form von O/W-Emulsionen, W/O-Emulsionen, O/W/O-Emulsionen, W/O/W-Emulsionen, O/W-Mikroemulsion, W/O-Mikroemulsionen, wässrigen, alkoholischen oder wässrig-alkoholischen Gelen sowie als Öl vorliegen können, und aus Rinse-off-Produkten wie Waschgelen, Waschcremes, Duschgelen, Duschbädern, Seifen, Shampoos, Haarkonditioniermitteln, Haarfärbezusammensetzungen, Haarwell- und Haarglättungsmitteln sowie Tränkemulsionen und -lösungen für feste Substrate zur Reinigung und/oder Pflege der Haut und/oder der Haare.

8. Verwendung gemäß einem der Ansprüche 1 bis 7, **dadurch gekennzeichnet, dass** die topischen kosmetischen oder pharmazeutischen Zusammensetzungen weiterhin mindestens einen Wirkstoff, ausgewählt aus anorganischen und organischen UV-Filtersubstanzen, Vitaminen, Provitaminen und Vitaminvorstufen der Gruppen A, B, C, E, H und K oder deren Derivaten, Allantoin, $\alpha$-Bisabolol, $\alpha$-Liponsäure, $\alpha$-Hydroxycarbonsäuren sowie Desoxyzuckern oder Desoxyzucker-Bausteine enthaltenden Polysacchariden, enthalten.

9. Topische kosmetische oder pharmazeutische Zusammensetzungen mit entzündungshemmender Wirkung mit einem Gehalt an 6,7-disubstituierten 2,2-Dialkylchromanen oder -chromenen der allgemeinen Formeln (I) oder (II),

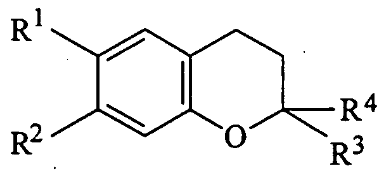

(I)

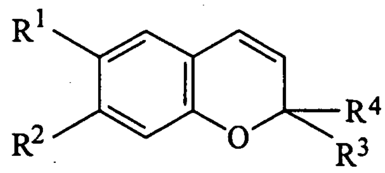

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen,
**gekennzeichnet durch** einen Gehalt an einem weiteren Wirkstoff, ausgewählt aus :

- den Vitaminen, Provitaminen und Vitaminvorstufen der Gruppen A, B, C, E, H und K oder deren Derivaten,
- α-Bisabolol,
- α-Liponsäure,
- α-Hydroxycarbonsäuren sowie
- Desoxyzuckern oder Desoxyzucker-Bausteine enthaltenden Polysacchariden.

Europäisches Patentamt

# EUROPÄISCHER RECHERCHENBERICHT

Nummer der Anmeldung
EP 03 00 2727

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 01 82888 A (CALS GRIERSON MARIE MADELEINE ;PELLETIER PASCALE (FR); OREAL (FR)) 8. November 2001 (2001-11-08)<br>* Seite 9, Zeile 25-32; Ansprüche 1,9 * | 1-9 | A61K7/48<br>A61K7/02 |
| P,X<br><br><br>P,A | WO 02 49593 A (HENKEL KGAA ;FOERSTER THOMAS (DE); PETERSOHN DIRK (DE); SCHLOTMANN) 27. Juni 2002 (2002-06-27)<br>* Ansprüche 13,14 * | 9<br><br><br>1-8 | |
| A | PATENT ABSTRACTS OF JAPAN<br>vol. 2000, no. 16,<br>8. Mai 2001 (2001-05-08)<br>& JP 2001 002558 A (KANEBO LTD),<br>9. Januar 2001 (2001-01-09)<br>* Zusammenfassung * | 1-9 | |
| A | DE 100 20 447 A (HENKEL KGAA)<br>11. Oktober 2001 (2001-10-11)<br>* Seite 6, Zeile 42-62; Ansprüche 1-8 * | 1-9 | RECHERCHIERTE SACHGEBIETE (Int.Cl.7)<br>A61K |

Der vorliegende Recherchenbericht wurde für alle Patentansprüche erstellt

| Recherchenort | Abschlußdatum der Recherche | Prüfer |
|---|---|---|
| MÜNCHEN | 26. Mai 2003 | Lindner, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 03 00 2727

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-05-2003

| Im Recherchenbericht angeführtes Patentdokument | Datum der Veröffentlichung | Mitglied(er) der Patentfamilie | Datum der Veröffentlichung |
|---|---|---|---|
| WO 0182888 A | 08-11-2001 | FR 2808189 A1 | 02-11-2001 |
| | | EP 1278509 A1 | 29-01-2003 |
| | | WO 0182888 A1 | 08-11-2001 |
| WO 0249593 A | 27-06-2002 | DE 10063433 A1 | 27-06-2002 |
| | | AU 3841802 A | 01-07-2002 |
| | | WO 0249593 A2 | 27-06-2002 |
| JP 2001002558 A | 09-01-2001 | KEINE | |
| DE 10020447 A | 11-10-2001 | DE 10020447 A1 | 11-10-2001 |
| | | AU 4250201 A | 15-10-2001 |
| | | WO 0174320 A2 | 11-10-2001 |
| | | EP 1267813 A2 | 02-01-2003 |

EPO FORM P0461

Für nähere Einzelheiten zu diesem Anhang : siehe Amtsblatt des Europäischen Patentamts, Nr.12/82